(19) Europäisches Patentamt
European Patent Office
Office européen des brevets

(11) Veröffentlichungsnummer: **0 080 555**
**B1**

# (12) EUROPÄISCHE PATENTSCHRIFT

(45) Veröffentlichungstag der Patentschrift: **07.01.87**

(51) Int. Cl.⁴: **C 07 D 235/32**, C 07 D 235/30

(21) Anmeldenummer: **82106804.6**

(22) Anmeldetag: **17.10.80**

(60) Veröffentlichungsnummer der früheren Anmeldung nach Art. 76 EPÜ: **0027646**

## (54) Verfahren zur Herstellung von 5(6)-Thiocyano-benzimidazol-Derivaten.

(30) Priorität: **19.03.80 HU 63580**

(43) Veröffentlichungstag der Anmeldung: **08.06.83 Patentblatt 83/23**

(45) Bekanntmachung des Hinweises auf die Patenterteilung: **07.01.87 Patentblatt 87/2**

(84) Benannte Vertragsstaaten: **AT CH DE FR GB IT LI**

(56) Entgegenhaltungen:
**EP - A - 0 003 951**
**DE - A - 2 041 324**
**DE - A - 2 363 348**
**DE - A - 2 454 632**
**US - A - 4 002 640**

(73) Patentinhaber: **CHINOIN Gyogyszer és Vegyészeti Termékek Gyára RT., To utca 1-5, H-1045 Budapest IV (HU)**

(72) Erfinder: **Gönczi, Csaba, Dr. Dipl.Ing.Chem., Lisznyai u.5, H-1016 Budapest (HU)**
Erfinder: **Korbonits, Dezsö, Dr. Dipl.Ing.Chem., Vérhalom u.27/D, H-1025 Budapest (HU)**
Erfinder: **Pálosi, Endre, Dipl.Ing.Chem., Pasaréti ut 114/a, H-1026 Budapest (HU)**
Erfinder: **Kiss, Pál, Dr.Dipl.Ing.Chem., Vetés u.82, H-1046 Budapest (HU)**
Erfinder: **Héja, Gergely, Dr. Dipl.Ing.Chem., Sollner u.27, H-1131 Budapest (HU)**
Erfinder: **Kun, Judit, Dipl.Ing.Chem., Gyenes u.17, H-1032 Budapest (HU)**
Erfinder: **Szomor geb.Wundele, Mária, Dipl.Ing.Chem., Fö u.21, H-1011 Budapest (HU)**
Erfinder: **Szvoboda geb.Kanzel, Ida, Dipl.Ing.Chem., Fogarasi u.85 1/5, H-1141 Budapest (HU)**
Erfinder: **Márványos, Ede, Dipl.Ing.Chem., Wesselényi u.69, H-1077 Budapest (HU)**
Erfinder: **Kovács geb.Mindler, Vera, Dipl.Ing.Chem., Rná Park 4, H-1142 Budapest (HU)**

(74) Vertreter: **Lotterhos, Hans Walter, Dr.-Ing., Lichtensteinstrasse 3, D-6000 Frankfurt am Main 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

**Beschreibung**

Die Erfindung betrifft ein Verfahren zur Herstellung von 5(6)-Thiocyano-benzimidazol-Derivaten der allgemeinen Formel I

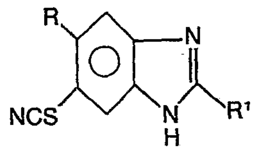

(I)

worin

R Wasserstoff, Halogen, $C_{1-4}$-Alkyl, $C_{1-3}$-Alkoxy oder Trifluormethyl,

$R^1$ Amino oder $C_{1-4}$-Alkoxycarbonylamino bedeuten,

sowie deren Salzen.

Die 5(6)-Thiocyano-benzimidazol-Derivate der allgemeinen Formel I stellen Zwischenprodukte für die Herstellung der als Anthelmintica bekannten Benzimidazolderivate der allgemeinen Formel

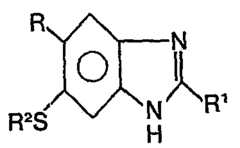

dar,

worin

R und $R^1$ die oben angegebene Bedeutung haben und

$R^2$ ein ionisch gebundenes Metallatom, ein ionisch oder kovalent gebundenes Wasserstoffatom, $C_{1-6}$-Alkyl, Alkenyl oder Alkinyl mit jeweils bis zu 6 C-Atomen, oder Cyclohexyl, Benzyl oder 2-Phenyläthyl bedeutet,

wobei ein 5(6)-Thiocyano-benzimidazol-Derivat der allgemeinen Formel I durch Behandeln mit einer nucleophilen, organischen oder anorganischen Base in eine Verbindung der allgemeinen Formel

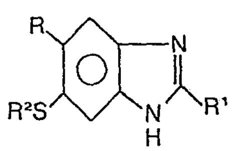

worin

R und $R^1$ die oben angegebene Bedeutung haben und

$R^2$ ein ionisch gebundenes Metallatom, ein ionisch oder kovalent gebundenes Wasserstoffatom bedeutet,

übergeführt wird, die gegebenenfalls mit einem Alkylierungsmittel der allgemeinen Formel

$$R^{2''} - Q,$$

worin

$R^2$ $C_{1-6}$-Alkyl, Alkenyl oder Alkinyl mit jeweils bis zu 6 C-Atomen, Cyclohexyl, Benzyl oder 2-Phenyläthyl und

Q Hydroxy, Halogen, ½ $SO_4$, ½ $PO_3$, ⅓ $PO_4$, $SO_3C_6H_5$ oder $SO_3C_6H_4CH_3$ bedeuten,

umgesetzt wird.

Nach der US-PS 4 080 461 ist es bereits bekannt, zur Herstellung von 5(6)-Thiocyano-benzimidazol-Derivaten der allgemeinen Formel I 1-Amino-2-nitro-4-thiocyano-benzol zunächst zum 1,2-Diamino-4-thiocyano-benzol zu reduzieren und dann durch Umsetzen mit 1,2-Bis-(methoxycarbonyl)-S-methyl-isothioharnstoff unter Bildung des Benzimidazolringes zu cyclisieren. Dieses Verfahren ist jedoch nicht in industriellem Massstab durchführbar. Die Reduktion zum 1,2-Diamino-4-thiocyanobenzol muss in konzentrierter Salzsäure mit grossem Überschuss an Zinn(II)-chlorid bei −40 °C (US-PS 4 080 461, Beispiel 1) durchgeführt werden, und die Cyclisierung mit 1,3-Bis-(methoxycarbonyl)-S-methyl-isothioharnstoff verläuft unter Mercaptanentwicklung, was mit einer Umweltbelastung verbunden ist.

Es wurde gefunden, dass die 5(6)-Thiocyano-benzimidazol-Derivate der allgemeinen Formel I durch selektive Rhodanierung, d.h. durch Umsetzen der Verbindungen der allgemeinen Formel II

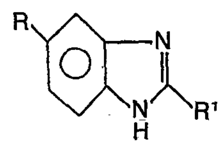

(II)

in der R und $R^1$ die oben angegebene Bedeutung haben,

mit Chlorgas und einem Rhodanid der allgemeinen Formel III

$$R^3 - SCN \qquad (III)$$

worin

$R^3$ ein Alkalimetall- oder Erdalkalimetallatom oder die Ammoniumgruppe bedeutet,

in grosser Reinheit und hoher Ausbeute hergestellt werden können. Die Verfahrensführung dieses Verfahrens ist sehr einfach, so dass sich das Verfahren auch in industriellem Massstab durchführen lässt.

Die direkte Rhodanierung der 2-Benzimidazolyl-carbamate der allgemeinen Formel II ist bislang nicht bekannt geworden. In der Literatur [z. B. S. Patai: «The Chemistry od Cyanates and their Thio Derivates», Teil 2, S. 837 (1977), Wiley; Pharmazie 32 (4), 195 (1977)] ist zwar die Rhodanierung von zahlreichen heterocyclischen Verbindungen beschrieben, nicht aber die von Benzimidazol-derivaten. Desgleichen ist bislang nichts über die Halogenierung von 2-Benzimidazolylcarbamaten – die Halogenierung ist bekanntlich eine Reaktion, die der Rhodanierung chemisch sehr nahe steht – in der Literatur bekannt geworden. Dies dürfte darauf beruhen, dass man einerseits mit der Zersetzung der sehr empfindlichen Carbamatgruppe und andererseits mit der Bildung von unerwünschten 4(7)-substituierten Nebenprodukten rechnen musste, da die Halogenierung von 2-substituierten Benzimidazolen zu einem Gemisch von 4(7)-Halogen- und 4(7), 5(6)-Dihalogenprodukten geführt hatte [Chem. Rev. 48, 490 (1951)].

Es war daher sehr überraschend, dass die direkte Rhodanierung von 2-Benzimidazolyl-carbamaten der allgemeinen Formel II selektiv zu den

5(6)-Thiocyano-Derivaten der allgemeinen Formel I führt, die in 90%iger Ausbeute und 99%igem Reinheitsgrad erhalten werden. Auf Grund des hohen Reinheitsgrads können die so erhaltenen 5(6)-Thiocyano-benzimidazol-Derivate der allgemeinen Formel I ohne weitere Reinigung zu den weiter oben angegebenen Anthelmintica weiter verarbeitet werden.

Nach einer vorteilhaften Ausführungsform des Verfahrens wird als Rhodanierungsreagens Chlorrhodan verwendet, das aus einem Rhodanid der allgemeinen Formel III und Chlorgas gebildet wird. Zweckmässig wird so verfahren, dass man das Chlorrhodan durch Reaktion eines Rhodanids der allgemeinen Formel III mit Chlorgas in einem wasserfreien organischen Lösungsmittel herstellt und danach einer Lösung der Verbindung der allgemeinen Formel II in einem organischen Lösungsmittel zufügt. Man kann aber auch die Lösung der Verbindung der allgemeinen Formel II der Chlorrhodanlösung zugeben.

Das Verfahren kann auch so durchgeführt werden, dass das Chlorgas in eine mit einem organischen Lösungsmittel hergestellte Lösung oder Suspension der Verbindungen der allgemeinen Formeln II und III eingeleitet wird. In diesem Fall reagiert das im statu nascendi befindliche Chlorrhodan sogleich mit der Verbindung der allgemeinen Formel II.

In dem Verfahren der Erfindung werden die Verbindungen der allgemeinen Formel II und das Chlorrhodan im Molverhältnis von 1:1 bis 1:5, vorzugsweise 1:1 bis 1:3 eingesetzt.

Nach einer anderen vorteilhaften Ausführungsform des Verfahrens werden einer mit einem wasserfreien organischen Lösungsmittel oder Lösungsmittelgemisch gebildeten Lösung von 1 Mol Verbindung der allgemeinen Formel II, 1 bis 3 Mol Rhodanid der allgemeinen Formel III zugesetzt und danach in die Lösung bei Temperaturen zwischen 15 und 40 °C 1 bis 4 Mol trockenes Chlorgas eingeleitet.

Der Lösung oder Suspension der Verbindungen der allgemeinen Formeln II und III kann das Chlorgas auch in Form einer mit einem organischen Lösungsmittel gebildeten Lösung zugefügt werden. Das Chlorgas oder dessen Lösung wird vorzugsweise innerhalb kurzer Zeit zugeführt.

Als Rhodanide der allgemeinen Formel III können Alkalimetall-, Erdalkalimetall- oder Ammoniumrhodanid verwendet werden, wobei Kaliumrhodanid, Natriumrhodanid oder Ammoniumrhodanid der Vorzug gegeben wird.

Als organische Lösungsmittel und/oder Verdünnungsmittel können dipolare, aprotische Lösungsmittel, in denen die Komponenten gut lösbar sind, wie Dimethylformamid, Dimethylsulfoxyd, Hexamethylphosphorsäuretriamid, niedrige Carbonsäuren, Ester, insbesondere Acetate oder Formiate, $C_{1-4}$-Alkohole, chlorierte Kohlenwasserstoffe, wie $CCl_4$, Chloroform, Dichlormethan oder Dichloräthan, weiterhin Nitromethan oder Acetonitril verwendet werden. Hiervon wird insbesondere Essigsäure bevorzugt.

Die Reaktion wird bei Temperaturen zwischen 0 und 100 °C, vorzugsweise zwischen 15 und 40 °C, durchgeführt.

Weitere Einzelheiten des Verfahrens der Erfindung sind den nachstehenden Beispielen zu entnehmen.

Beispiel 1

In eine Lösung von 5 g 2-(Methoxycarbonyl-amino)-benzimidazol und 7,5 g trockenem Kaliumrhodanid in 100 ml Essigsäure werden 5,5 g trockenes Chlorgas eingeleitet. Das so erhaltene Reaktionsgemisch wird mit 100 ml Wasser verdünnt, der pH-Wert der Lösung mit 5 n Natriumhydroxydlösung auf 4 eingestellt, der ausgefallene Niederschlag abgesaugt, mit Wasser gewaschen und bei 60 °C getrocknet.

Es werden 5,6 g (90% Ausbeute) 5(6)-Thiocyano-2-(methoxycarbonyl-amino)-benzimidazol erhalten; Fp.: 252 bis 254 °C (unter Zersetzung).

Beispiel 2

70 ml Tetrachlorkohlenstoff, der 5,32 g Chlor enthält, versetzt man unter Rühren bei 10 bis 15 °C mit einer Lösung von 7,5 g trockenem Kaliumrhodanid in 60 ml wasserfreier Essigsäure. Das so erhaltene Reaktionsgemisch wird noch weitere 15 Minuten gerührt und danach mit 5 g 2-(Methoxycarbonyl-amino)-benzimidazol in 25 ml wasserfreier Essigsäure versetzt. Nach Rühren bei Zimmertemperatur wird das Reaktionsgemisch in Wasser gegossen, die Tetrachlorkohlenstoffphase abgetrennt und nach Entfärbung mit Aktivkohle der pH-Wert der wässrigen Lösung mit 5 n Natriumhydroxydlösung auf 4 eingestellt. Der ausgefallene Niederschlag wird abgesaugt, mit Wasser gewaschen und bei 60 °C getrocknet. So erhält man 5(6)-Thiocyano-2-(methoxycarbonyl-amino)-benzimidazol; Fp.: 248 bis 250 °C (unter Zersetzung).

**Patentansprüche**

1. Verfahren zur Herstellung von 5(6)-Benzimidazol-Derivaten der allgemeinen Formel I

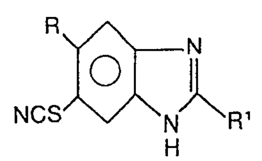

(I)

worin

R Wasserstoff, Halogen, $C_{1-4}$-Alkyl, $C_{1-3}$-Alkoxy oder Trifluormethyl,

$R^1$ Amino oder $C_{1-4}$-Alkoxycarbonylamino bedeuten, sowie deren Salzen,

dadurch gekennzeichnet, dass man eine Verbindung der allgemeinen Formel II

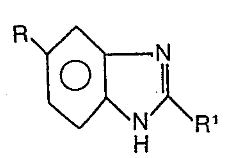

(II)

in der R und $R^1$ die oben angegebene Bedeutung haben,
mit Chlorgas und einem Rhodanid der allgemeinen Formel III

$$R^3 - SCN \qquad (III)$$

worin
$R^3$ ein Alkalimetall- oder Erdalkalimetallatom oder die Ammoniumgruppe bedeutet,
umsetzt.

2. Verfahren nach Anspruch 1, dadurch gekennzeichnet, dass man zur Rhodanierung des Benzimidazolderivats der allgemeinen Formel II entweder Chlorgas in eine Lösung oder Suspension der Verbindungen der allgemeinen Formeln II und III einleitet oder die Verbindungen der allgemeinen Formel II mit dem aus einem Rhodanid der allgemeinen Formel III und Chlorgas erhaltenen Chlorrhodan umsetzt.

3. Verfahren nach Anspruch 1 oder 2, dadurch gekennzeichnet, dass man als Rhodanid der allgemeinen Formel III Kalium- oder Natriumrhodanid verwendet.

**Claims**

1. Process for the preparation of 5(6)-benzimidazole derivatives of general formula I

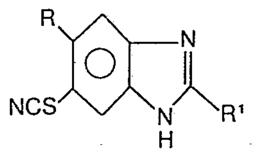

(I)

wherein
R represents hydrogen, halogen, $C_{1-4}$ alkyl, $C_{1-3}$ alkoxy or trifluoromethyl,
$R^1$ represents amino or $C_{1-4}$ alkoxycarbonylamino, and the salts thereof,
characterised in that a compound of general formula II

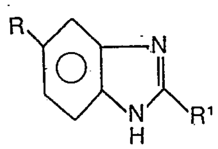

(II)

wherein R and $R^1$ are as hereinbefore defined, is reacted with chlorine gas and a rhodanide of general formula III

$$R^3 - SCN \qquad (III)$$

wherein
$R^3$ represents an alkali metal or alkaline earth metal atom or the ammonium group.

2. Process as claimed in claim 1, characterised in that, in order to rhodanise the benzimidazole derivative of general formula II, either chlorine gas is introduced into a solution or suspension of the compounds of general formulae II and III or the compounds of general formula II are reacted with the rhodanic chloride obtained from a rhodanide of general formula III and chlorine gas.

3. Process as claimed in claim 1 or 2, characterised in that potassium or sodium rhodanide is used as the rhodanide of general formula III.

**Revendications**

1. Procédé pour la préparation de dérivés de thiocyano-5(6)benzimidazole de formule générale I

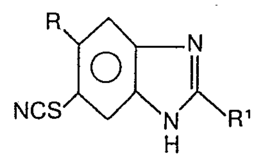

(I)

dans laquelle
R représente l'hydrogène, un halogène, un alkyle en $C_1$ à $C_4$, un alcoxy en $C_1$ à $C_3$ ou un trifluorométhyle,
$R^1$ représente un amino ou un alcoxy $C_{1-4}$ carbonylamino, ainsi que de leurs sels,
caractérisé en ce que l'on fait réagir un composé de formule générale II

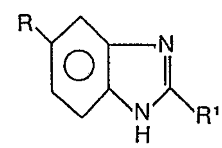

(II)

dans laquelle R et $R^1$ ont la signification précédente, avec du chlore gazeux et un thiocyanate de formule générale III

$$R^3 - SCN \qquad (III)$$

dans laquelle
$R^3$ représente un atome de métal alcalin ou de métal alcalino-terreux ou le groupe ammonium.

2. Procédé selon la revendication 1, caractérisé en ce que, pour la thiocyanuration du dérivé de benzimidazole de formule générale II, on fait passer du chlore gazeux dans une solution ou une suspension des composés de formules générales II et III ou fait réagir les composés de formule générale II avec le chlorothiocyanogène obtenu à partir d'un thiocyanate de formule générale III et de chlore gazeux.

3. Procédé selon la revendication 1 ou 2, caractérisé en ce qu'on emploie comme thiocyanate de forme générale III du thiocyanate de potassium ou de sodium.